(19) Europäisches Patentamt
European Patent Office
Office européen des brevets

(11) **EP 4 521 099 A1**

(12) **EUROPEAN PATENT APPLICATION**

(43) Date of publication:
**12.03.2025 Bulletin 2025/11**

(21) Application number: **24198598.5**

(22) Date of filing: **05.09.2024**

(51) International Patent Classification (IPC):
*G01N 21/64* (2006.01)   *G01N 15/00* (2024.01)
*G01N 21/77* (2006.01)

(52) Cooperative Patent Classification (CPC):
**G01N 21/6445; G01N 15/0211;** G01N 2015/0053;
G01N 2015/0222; G01N 2021/7786

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **11.09.2023 JP 2023147242**

(71) Applicants:
• **CANON KABUSHIKI KAISHA**
**Tokyo 146-8501 (JP)**
• **Canon Medical Systems Corporation**
**Tochigi 324-0036 (JP)**

(72) Inventors:
• **KAKEGAWA, Norishige**
**Tokyo (JP)**
• **MASUMURA, Takahiro**
**Tokyo (JP)**
• **SAKAKIBARA, Teigo**
**Tokyo (JP)**
• **KOMORI, Yuya**
**Tokyo (JP)**

(74) Representative: **WESER & Kollegen**
**Patentanwälte PartmbB**
**Radeckestraße 43**
**81245 München (DE)**

(54) **ANALYSIS METHOD, ANALYSIS APPARATUS, AND ANALYSIS KIT FOR CALCULATING CONCENTRATION OF TARGET SUBSTANCE**

(57)    Provided is an analysis method for determining at least any one of: presence or absence of a target substance; and a concentration of the target substance through use of a reagent that reacts with the target substance, the analysis method including: a loading step of loading: a sample containing the target substance; a hydrophilic polymer that reacts with a silanol group of a glass vessel; and the reagent into the glass vessel; a reaction step of causing the target substance and the reagent to react with each other to provide a reaction liquid; and an analysis step of determining at least any one of: the presence or absence of the target substance; and the concentration of the target substance in the reaction liquid, wherein the loading step includes loading the reagent after or simultaneously with the loading of the hydrophilic polymer.

FIG. 1

LOADING STEP

REACTION STEP

ANALYSIS STEP

REMOVAL STEP

WASHING STEP

EP 4 521 099 A1

**Description**

BACKGROUND OF THE INVENTION

Field of the Invention

The present invention relates to an analysis method, an analysis apparatus, and an analysis kit for calculating the concentration of a target substance.

Description of the Related Art

In the fields of medicine and clinical tests, high-sensitivity detection or quantification of a trace amount of a biological component from, for example, blood or a collected part of an organ is required for investigating, for example, the cause and presence or absence of a disease. Among test techniques for biological components, immunoassays are widely utilized. In many of the immunoassays, a washing step called bound/free (B/F) separation is required. As an immunoassay that does not require the B/F separation, there is given a latex agglutination method utilizing an antigen-antibody reaction. In the latex agglutination method, latex particles each supporting thereon, for example, an antibody that specifically binds to a target substance are mixed with a liquid that may contain the target substance, and the degree of agglutination of the latex particles is measured.

In the latex agglutination method, the target substance is captured by the antibody bound to the latex particles and specific to the target substance, and a plurality of the latex particles are crosslinked via the captured target substance, with the result that the agglutination of the latex particles occurs. That is, the amount of the target substance in a liquid sample such as a biological sample can be quantified by evaluating the degree of the agglutination of the latex particles. The degree of the agglutination can be quantified by measuring and evaluating a change in amount of light transmitted through or scattered by the liquid sample.

The latex agglutination method can detect/quantitatively evaluate an antigen as the target substance in a simple and rapid manner. Meanwhile, the latex agglutination method has involved a problem with detection limits in that the antigen cannot be detected when its amount in the liquid sample such as the biological sample is small.

In order to improve detection sensitivity for the target substance, it is required that the degree of the agglutination be measured with higher sensitivity. That is, it is conceivable to replace a system for measuring the change in amount of the light transmitted through or scattered by the liquid sample with a method for detection/quantification utilizing a luminescence characteristic with higher sensitivity. Specifically, there has been proposed, for example, a specimen test method utilizing a fluorescence depolarization method (Japanese Patent Publication No. H03-52575 and Japanese Patent No. 2893772). In Japanese Patent Publication No. H03-52575, it is proposed that an apparatus for the fluorescence depolarization method be improved to be clinically used.

In the fluorescence depolarization method, the B/F separation required in a general fluorescence measurement method is not required. Accordingly, use of the fluorescence depolarization method enables a simple specimen test as with the latex agglutination method. Further, it is conceived that use of the fluorescence depolarization method enables measurement by the same test system as that in the latex agglutination method by merely mixing a luminescent substance that specifically reacts with the target substance in a measurement process. Meanwhile, in Japanese Patent Publication No. H03-52575, there is a proposal of use of a single molecule such as fluorescein as a luminescent material, which is applicable only to a drug, a low-molecular-weight antigen, and the like in principle.

Japanese Patent No. 2893772 has solved the problem of Japanese Patent Publication No. H03-52575, i.e., the problem in that the fluorescence depolarization method is applied only to a drug, a low-molecular-weight antigen, and the like. That is, in Japanese Patent No. 2893772, with an aim to apply the fluorescence depolarization method to a macromolecule such as a protein, it is proposed to use, as a luminescent material, a material obtained by causing a dye having a long-lifetime luminescence characteristic to adsorb to latex particles. In Japanese Patent No. 2893772, it is proposed that a high-molecular-weight substance be quantified by balancing a reduction in rotational Brownian motion of the substance in a liquid due to an increase in particle diameter and the length of emission lifetime based on the principle of the fluorescence depolarization method. In the fluorescence depolarization method using such particles, it is required to reduce signal noise by subjecting the particles to surface treatment to make the surfaces of the particles hydrophilic, to thereby suppress nonspecific adsorption.

When high-sensitivity measurement is performed by using measurement based on the fluorescence depolarization method, it is required to accurately measure a slight difference in luminescence amount between the respective polarized light components. Accordingly, it is essential to remove a factor for the signal noise for performing the high-sensitivity measurement.

One of such signal noise was a problem occurring when an optical cell for measuring the polarized light component was reutilized. In general, when the optical cell made of glass is used for measurement, the optical cell can be repeatedly used by being washed after its use. Accordingly, the use of the optical cell made of glass is suitable in terms of cost. However, when washing of the optical cell after measurement of a sample is insufficient, and the luminescent material used for the measurement remains on the wall surface of the cell even in a slight amount, accurate spectroscopic measurement cannot be performed at the time of measurement of the next sample.

In particular, in measurement based on the fluorescence depolarization method aimed at the high-sensitivity measurement, high-accuracy washing may even become a factor for determining the sensitivity limits of a measurement method.

In particular, an affinity between each of the particles whose surfaces are modified to be hydrophilic and a silanol group present on a glass surface may be increased, with the result that the particles each have a high possibility of adhering to the wall surface of the cell.

SUMMARY OF THE INVENTION

There is provided an analysis method capable of reducing noise at the time of calculation of the concentration of a target substance through use of a reagent that reacts with the target substance, particularly a method, such as a method based on polarization anisotropy, capable of reducing noise by suppressing nonspecific adsorption of the reagent onto a glass vessel when high-sensitivity measurement is performed through use of a small amount of luminescent particles.

As one embodiment, the present invention provides an analysis method for determining at least any one of: presence or absence of a target substance; and a concentration of the target substance through use of a reagent that reacts with the target substance, the analysis method including: a loading step of loading: a sample containing the target substance; a hydrophilic polymer that reacts with a silanol group of a glass vessel; and the reagent into the glass vessel; a reaction step of causing the target substance and the reagent to react with each other to provide a reaction liquid; and an analysis step of determining at least any one of: the presence or absence of the target substance; and the concentration of the target substance in the reaction liquid, wherein the reagent includes a luminescent reagent having a hydrophilic surface, and the luminescent reagent includes a luminescent particle, and wherein the loading step includes loading the reagent after or simultaneously with the loading of the hydrophilic polymer.

In addition, as one embodiment, the present invention provides an analysis apparatus for determining at least any one of presence or absence of a target substance; and a concentration of the target substance, the analysis apparatus including: a glass vessel to be used for accommodating a reaction liquid containing: a sample containing the target substance; and a reagent that reacts with the target substance; a unit configured to load the sample containing the target substance into the glass vessel; a unit configured to load a hydrophilic polymer that reacts with a silanol group of the glass vessel into the glass vessel; a unit configured to load the reagent into the glass vessel after or simultaneously with the loading of the hydrophilic polymer; an irradiation unit configured to irradiate, as irradiation light, the glass vessel with light that is linearly polarized light; a measurement unit configured to measure fluorescence intensities of fluorescence of two polarized light components in a parallel direction and an orthogonal direction with respect to the linearly polarized light; and a processing unit configured to process results obtained by the measurement unit, to thereby determine at least any one of the presence or absence of the target substance; and the concentration of the target substance in the reaction liquid, wherein the reagent includes a luminescent reagent having a hydrophilic surface, and the luminescent reagent includes a luminescent particle.

In addition, as one embodiment, the present invention provides an analysis kit to be used in an analysis method for determining at least any one of presence or absence of a target substance; and a concentration of the target substance based on polarization anisotropy, the analysis kit including: a reagent that reacts with the target substance; a glass vessel to be used for accommodating a reaction liquid containing: a sample containing the target substance; and the reagent; and a hydrophilic polymer that reacts with a silanol group of the glass vessel, wherein the reagent includes a luminescent reagent having a hydrophilic surface, and the luminescent reagent includes a luminescent particle.

Further features of the present invention will become apparent from the following description of exemplary embodiments with reference to the attached drawings.

BRIEF DESCRIPTION OF THE DRAWINGS

FIG. 1 is a schematic view for illustrating an analysis method according to one embodiment of the present invention.
FIG. 2 is a schematic view for illustrating an analysis apparatus according to one embodiment of the present invention.
FIG. 3 is a schematic view for illustrating part of the analysis apparatus according to one embodiment of the present invention.
FIG. 4 is a schematic view for illustrating a luminescent reagent to be used in one embodiment of the present invention.

DESCRIPTION OF THE EMBODIMENTS

Exemplary embodiments of the present invention are described in detail below. However, the embodiments are not intended to limit the scope of the present invention.

The present invention provides the following analysis method as one embodiment.

The analysis method according to one embodiment of the present invention is an analysis method for determining at least any one of: presence or absence of a target substance; and a concentration of the target substance through use of a reagent that reacts with the target substance, the analysis method including: a loading step of loading: a sample containing the target substance; a hydrophilic polymer that reacts with a silanol group of a glass vessel; and the reagent into the glass vessel; a reaction step of causing the target substance and the reagent to react with each other to provide a reaction liquid; and an analysis step of determining at least any one of: the presence or absence of the target substance; and the concentration of the target substance in the reaction liquid, wherein the reagent includes a luminescent reagent having a hydrophilic surface, and the luminescent reagent includes a luminescent particle, and wherein the loading step includes loading the reagent after or simultaneously with the loading of the hydrophilic polymer.

Herein, a polymer having a pyrrolidone ring, such as polyvinylpyrrolidone, is hereinafter sometimes abbreviated as "PVP".

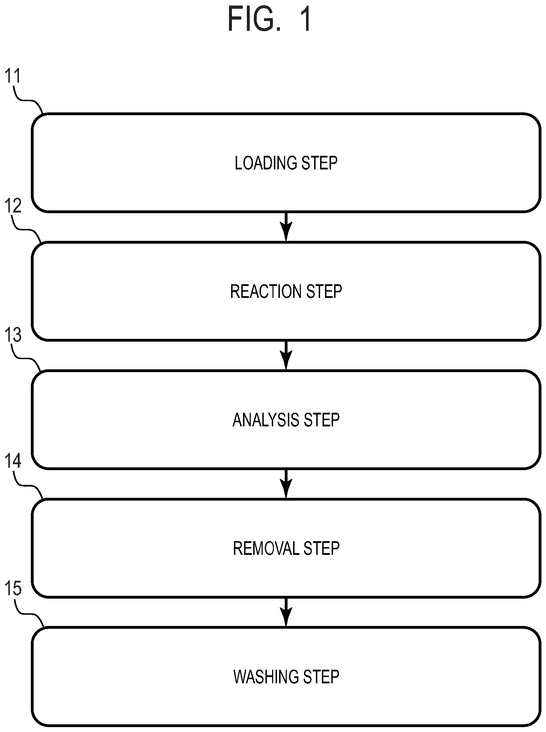

The analysis method according to one embodiment of the present invention may be described with reference to FIG. 1. That is, the analysis method according to one embodiment of the present invention includes a loading step 11, a reaction step 12, and an analysis step 13, and may further include a removal step 14 and a washing step 15. The steps are described below.


(With regard to Loading Step)

The analysis method according to one embodiment of the present invention includes the loading step of loading: a sample containing the target substance; a hydrophilic polymer that reacts with a silanol group of a glass vessel; and the reagent into the glass vessel. In the loading step, the reagent is loaded after or simultaneously with the loading of the hydrophilic polymer.

As long as the reagent is not loaded into the glass vessel before the hydrophilic polymer, the hydrophilic polymer, the sample, and the reagent may all be loaded simultaneously, or may be loaded separately from one another. In addition, as long as the reagent is not loaded into the glass vessel before the hydrophilic polymer, two kinds out of the hydrophilic polymer, the sample, and the reagent may be loaded simultaneously, and the other one kind may be loaded at a separate timing. Specifically, there are given: a method involving first loading the hydrophilic polymer, followed by loading the sample and the reagent simultaneously or separately from each other; and a method involving first loading the sample, followed by loading the hydrophilic polymer and the reagent simultaneously or by loading the reagent after loading the hydrophilic polymer. In addition, there are given: a method involving loading the hydrophilic polymer and the reagent simultaneously, followed by loading the sample; and a method involving loading the hydrophilic polymer and the sample simultaneously, followed by loading the reagent. When the sample and the reagent are loaded simultaneously, the sample and the reagent may be loaded after having been caused to react with each other in another vessel.

In the loading step, the hydrophilic polymer may be loaded into the glass vessel simultaneously with a diluent after having been dissolved in the diluent in advance. The diluent may serve as a solvent at the time of loading of the hydrophilic polymer into the glass vessel, and may contain the sample, the reagent, or any other substance.

When the concentration of the hydrophilic polymer with respect to the diluent is sufficient, a reaction between the silanol group present on the inner wall of the glass vessel and the hydrophilic polymer is started immediately after loading, and the inner wall of the glass vessel is coated with the hydrophilic polymer. The reaction between the hydrophilic polymer and the silanol group is, for example, a hydrogen bond or adsorption based on an electrostatic interaction. Even when the hydrophilic polymer and the reagent are loaded simultaneously, the luminescent reagent can be prevented from adsorbing onto the wall surface of the glass vessel. It is conceived that when the concentration of the hydrophilic polymer is set to be higher than that of the luminescent reagent, the hydrophilic polymer reacts with the silanol group on the wall surface of the glass vessel predominantly over the luminescent reagent. It is only required that the concentration of the hydrophilic polymer be 0.001 mass% or more and 2.0 mass% or less with respect to the reaction liquid in the analysis step described later. A concentration of the hydrophilic polymer of 0.03 mass% or more and 0.3 mass% or less with respect to the reaction liquid in the analysis step can be more suitably used.

The reagent may also be loaded into the glass vessel under the state in which the target substance has not been loaded into the glass vessel. The hydrophilic polymer that reacts with the silanol group has already adsorbed onto the wall surface of the glass vessel before the luminescent reagent reacts with the silanol group on the wall surface, and hence the luminescent reagent can be prevented from adsorbing onto the wall surface of the glass vessel. When the luminescent reagent adsorbs onto the wall surface of the glass vessel, its rotational motion is suppressed, which results in, for example, an increase in value (R) for polarization anisotropy described later, and thus serves as a factor for the occurrence of measurement noise. Accordingly, when the adsorption of the luminescent reagent onto the wall surface of the glass vessel is suppressed, the possibility of the occurrence of the measurement noise can be reduced.

The hydrophilic polymer, the target substance, the reagent, and the diluent are described later.

(With regard to Reaction Step)

The analysis method according to one embodiment of the present invention includes the reaction step of causing the target substance and the reagent to react with each other to provide a reaction liquid. The reaction liquid is a liquid containing: the sample containing the target substance; and the reagent, and serves as a target for which at least any one of: the presence or absence of the target substance; and the concentration of the target substance is determined in the analysis step. The reaction liquid may contain an additive or the like except for the sample containing the target substance and the reagent, or may contain the hydrophilic polymer. The loading step may include loading the sample containing the target substance and the reagent into the glass vessel to mix the materials, to thereby cause the target substance and the reagent to react with each other in the glass vessel to provide the reaction liquid. When the target substance and the reagent are caused to react with each other by loading the sample containing the target substance and the reagent into the glass vessel and mixing the materials with each other in the loading step, the loading step may be regarded as including the reaction step. The reaction is preferably performed under the state in which the reaction liquid has a pH in the range of 3.0 or more and 11.0 or less. In addition, a reaction temperature may fall within the range of from 20°C to 50°C. A reaction time period is set in view of, for example, the concentration of the target substance in the sample or an affinity between the target substance and the reagent, but is preferably from about 5 minutes to about 24 hours, more preferably from about 5 minutes to about 1 hour.

(With regard to Analysis Step)

The analysis method according to one embodiment of the present invention includes the analysis step of determining at least any one of the presence or absence of the target substance; and the concentration of the target substance in the reaction liquid.

In the analysis step, at least any one of: the presence or absence of the target substance; and the concentration of the target substance in the reaction liquid may be determined by measuring a value (R) for polarization anisotropy of the reaction liquid. At this time, $\Delta R$ may be determined by measuring the R of the reaction liquid immediately after mixing of the sample containing the target substance and the reagent, and subtracting the resultant R from the R of the reaction liquid measured after performing the reaction for a certain time period. The presence or absence of the target substance or the concentration thereof in the reaction liquid may be obtained by comparing the magnitude of the resultant $\Delta R$ to $\Delta R$ measured for a liquid having a known concentration of the target substance in the same manner in advance.

Alternatively, the target substance may also be quantitatively evaluated by measuring the R of the reaction liquid every certain time period after the mixing of the sample containing the target substance and the reagent, plotting the Rs with respect to measurement times, and comparing the slopes.

Alternatively, the target substance may also be quantitatively evaluated by comparing the R of the reaction liquid after elapse of a certain time period from the mixing of the sample containing the target substance and the reagent.

Alternatively, the target substance may also be quantitatively evaluated by setting the R at the end point in advance, and comparing a time period required to reach the R.

In the analysis method according to one embodiment of the present invention, the presence or absence of the target substance may also be determined by comparing the concentration of the target substance to a predetermined threshold value. For example, the determination may be made in such a manner that when the concentration of the target substance is equal to or more than the predetermined threshold value, it is determined that the target substance is present, and when the concentration of the target substance is less than the predetermined threshold value, it is determined that the target substance is absent.

The measurement conditions are preferably as follows: for example, in the analysis step, the temperature of the reaction liquid is 0°C or more and 50°C or less, and the viscosity thereof is 0.5 mPa·s or more and 50 mPa·s or less. In the analysis step, the concentration of the luminescent reagent in the reaction liquid is preferably 0.0001 mg/ml or more and 0.1 mg/ml or less, and a detection wavelength is preferably 500 nm or more and 700 nm or less. In order to suppress the influence of multiple scattering, the optical path of an optical system may be shortened. For example, the optical path, which is generally set to 10 mm in many cases, may be set to 5 mm or less.

(With regard to Removal Step)

The analysis method according to one embodiment of the present invention may include the removal step of removing the reaction liquid after the analysis from the glass vessel. A removal method for the reaction liquid is not particularly limited, but a suction method can be suitably used. In addition, after the removal of the reaction liquid, it is preferred to promptly transfer to the washing step described below before a residual liquid, which fails to be removed, is dried.

(With regard to Washing Step)

The analysis method according to one embodiment of the present invention may include the washing step of washing the glass vessel after the analysis. In the washing step, the residual reaction liquid can be completely removed by rinsing the glass vessel with a washing liquid. Pure water may be used as the washing liquid. A solution in which a pH has been adjusted by adding an acidic or alkaline substance to pure water may also be used. In addition, the washing may be performed by repeatedly using pure water, an acidic liquid, and an alkaline liquid in sequence. A detergent that improves a washing effect, such as a surfactant, may also be added to the washing liquid. The acid, alkali, and detergent components can also be removed by repeatedly rinsing the glass vessel with pure water at the end of the washing step.

In order to improve the washing effect, the wall surface of the glass vessel may be rubbed with a sponge or the like that does not damage the glass vessel during a rinsing operation.

In order to improve the washing effect, the wall surface of the glass vessel may be vibrated by radiating an ultrasonic wave or the like thereto, to thereby generate bubbles in the liquid during the rinsing operation.

The hydrophilic polymer having adsorbed to the silanol group on the wall surface of the glass vessel does not need to be completely removed by the washing. It can be expected that the hydrophilic polymer exhibits a preventing effect on the adhesion of the luminescent reagent together with a hydrophilic polymer newly supplied at the time of analysis of the next sample.

In the analysis method according to one embodiment of the present invention, the luminescent reagent includes a particle, and hence the target substance, whose concentration is to be measured, can be detected in response to the aggregation/dispersion behavior of the particles. For example, in the analysis method for determining the presence or absence of the target substance or the concentration thereof based on polarization anisotropy, when the luminescent reagent includes the particle, a change in value (R) for polarization anisotropy can be grasped as a large one, and thus high-sensitivity measurement can be performed. Meanwhile, for example, when the luminescent reagent nonspecifically adsorbs onto the wall surface of the glass vessel at the time of measurement of the R, a numerical value higher than a proper value of the R that is supposed to be measured is measured. That is, the luminescent reagent having adsorbed becomes measurement noise. The luminescent reagent preferably has a hydrophilic surface property so that its nonspecific reaction with an impurity in the liquid is prevented. However, the luminescent reagent may form a hydrogen bond with the silanol group on the surface of the glass vessel owing to its hydrophilicity.

The inventors of the present invention have made investigations into reducing luminescence signal noise in the reaction between the target substance and the reagent at the time of repeated use of the glass vessel, to thereby perform high-sensitivity measurement, for example, measurement based on polarization anisotropy.

In order to reduce the luminescence signal noise, it is only required that the luminescent reagent equal to or higher than its measurement sensitivity be prevented from adsorbing onto the wall surface of the glass vessel.

That is, it is only required that an adsorption reaction occurring between the luminescent reagent and the silanol group of the glass vessel because the luminescent reagent has a hydrophilic surface be suppressed.

Particularly when high-sensitivity measurement using a small amount of the luminescent reagent is performed, its adsorption phenomenon onto the wall surface of the glass vessel prominently appears as noise. For example, when analysis based on polarization anisotropy is performed, the ratio of the aggregated luminescent reagent is observed before and after the reaction through use of the value (R) for polarization anisotropy. Accordingly, as the concentration of the luminescent reagent is set to be lower, a change in R observed along with its aggregation is increased more. That is, when high-sensitivity measurement is performed, the concentration of the luminescent reagent is preferably set to be lower. However, when the concentration of the luminescent reagent is low, the R changes (increases) even in a case in which the luminescent reagent adsorbs onto the wall surface of the glass vessel, which becomes noise at the time of measurement. Accordingly, the suppression of the adsorption of the luminescent reagent onto the inner wall surface of the glass vessel significantly contributes to the stability of the high-sensitivity measurement.

In the analysis method, an analysis apparatus, and an analysis kit according to one embodiment of the present invention, the surface of the glass vessel is coated with the hydrophilic polymer before the luminescent reagent reacts with the silanol group on the wall surface of the glass vessel. Accordingly, the adsorption of the luminescent reagent having a hydrophilic surface onto the surface of the glass vessel via the silanol group can be suppressed. As a result, a noise component, which occurs through the adsorption of the luminescent reagent onto the wall surface of the glass vessel, for example, an increase in value (R) for polarization anisotropy can be prevented.

In addition, it has been found that, according to the above-mentioned method, apparatus, and kit, the luminescent reagent can be sufficiently removed by a simple washing method at the time of washing of the glass vessel after the measurement.

(Value for Polarization Anisotropy)

In the analysis method according to one embodiment of the present invention, in the analysis step, at least any one of: the presence or absence of the target substance; and the concentration of the target substance in the reaction liquid may be determined by measuring a value (R) for polarization anisotropy of the reaction liquid. In one embodiment of the present invention, the value (R) for polarization anisotropy may be defined as described below. That is, the R may be set to a value showing a relationship between the luminescence intensity of a polarized light component in a parallel direction with respect to polarized light that is incident light and the luminescence intensity of a polarized light component in a perpendicular direction with respect thereto regarding luminescence generated by exciting a luminescent substance through irradiation with the polarized light. More specifically, the R may be set to a value calculated from the luminescence intensities of a luminescence component having a vibration direction parallel to that of given polarized light and a luminescence component having a vibration direction orthogonal (perpendicular) thereto, the luminescence intensities each being determined when the luminescent substance is excited by the polarized light. Further, the R may be set to a value indicating the ratio of a difference between the luminescence intensity of a luminescence component having a vibration direction parallel to that of a first polarized light beam at the time of excitation by the first polarized light beam and the luminescence intensity of a luminescence component having a vibration direction orthogonal to that of the first polarized light beam at the time of excitation by the first polarized light beam to the sum of the luminescence intensities.

The R may be corrected with: a ratio between the luminescence intensity of a luminescence component having a vibration direction orthogonal to that of a second polarized light beam having a vibration direction orthogonal to that of the first polarized light beam at the time of excitation by the second polarized light beam and the luminescence intensity of a luminescence component having a vibration direction parallel to that of the second polarized light beam having a vibration direction orthogonal to that of the first polarized light beam at the time of excitation by the second polarized light beam; and other constants. The value for polarization anisotropy encompasses values referred to as "polarization anisotropic property," "degree of polarization," and the like.

More specifically, for example, the R may be "r" in the following equation (1):

$$r = \frac{I_{vv} - G * I_{vH}}{I_{vv} + 2 * G * I_{vH}} \qquad (1)$$
$$G = \frac{I_{Hv}}{I_{HH}}$$

in the equation (1), $I_{VV}$ represents the luminescence intensity of a luminescence component having a vibration direction parallel to that of the first polarized light beam at the time of excitation by the first polarized light beam, $I_{VH}$ represents the luminescence intensity of a luminescence component having a vibration direction orthogonal to that of the first polarized light beam at the time of excitation by the first polarized light beam, $I_{HV}$ represents the luminescence intensity of a luminescence component having a vibration direction orthogonal to that of the second polarized light beam having a

vibration direction orthogonal to that of the first polarized light beam at the time of excitation by the second polarized light beam, $I_{HH}$ represents the luminescence intensity of a luminescence component having a vibration direction parallel to that of the second polarized light beam having a vibration direction orthogonal to that of the first polarized light beam at the time of excitation by the second polarized light beam, and G represents a correction value.

In addition, the R may be r' in the following equation (2):

$$r' = \frac{I_{vv} - G * I_{vH}}{I_{vv} + G * I_{vH}} \qquad (2)$$
$$G = \frac{I_{Hv}}{I_{HH}}$$

in the equation (2), each symbol is the same as that in the equation (1).

The measurement conditions for the R are as described below. For example, at the time of measurement, the temperature of the reaction liquid is preferably 0°C or more and 50°C or less, and the viscosity of the reaction liquid is preferably 0.5 mPa·s or more and 50 mPa·s or less. When the luminescent reagent includes a luminescent particle containing a europium complex, the concentration of the luminescent reagent in the reaction liquid is preferably 0.0001 mg/ml or more and 0.1 mg/ml or less, and a measurement wavelength is preferably 500 nm or more and 700 nm or less.

In addition, R0, which represents the R measured for the reagent unreacted with the target substance, preferably satisfies the formula: R0≥0.01.

(Diluent)

The diluent may include a water solvent as a main component. The pH of the diluent may be adjusted in order to promote the reaction between the target substance and the reagent. The pH may be adjusted to from 1.0 to 14.0 through use of a buffer solution. A pH of the diluent of from 5.0 to 9.0 can be suitably used. In addition, an additive may be added thereto in order to suppress the nonspecific adsorption and promote the reaction between the target substance and the reagent. However, it is required to select and use an additive that causes no interaction with each of the target substance and the reagent.

(Hydrophilic Polymer that reacts with Silanol Group)

A polymer having such physical properties that the polymer reacts with the silanol group present on the wall surface of the glass vessel and does not nonspecifically adsorb the luminescent reagent may be used as the hydrophilic polymer that reacts with a silanol group. The silanol group represents a structure in which a hydroxy group is directly bonded to silicon. The silanol group is also hydrophilic, and in an aqueous solution, the silanol group and a water molecule generally form a weak bond via a hydrogen bond in many cases. However, the silanol group has high reactivity, and may form a hydrogen bond with a molecule having a functional group except for water depending on conditions. This property causes the luminescent reagent to nonspecifically adsorb to the silanol group on the wall surface of the glass vessel. In order to prevent such phenomenon, it is effective to cause the hydrophilic polymer to adsorb to the silanol group on the wall surface of the glass vessel in advance.

For example, a hydrophilic polymer that forms a hydrogen bond with the silanol group of the glass vessel may be used as the hydrophilic polymer. It is required that the hydrophilic polymer that forms a hydrogen bond with the silanol group in one embodiment of the present invention have an ability to form a hydrogen bond equivalent to or stronger than an affinity between the luminescent reagent and the silanol group.

Specifically, for example, polyvinylpyrrolidone or polyethylene glycol can be suitably used.

In addition, a hydrophilic polymer that adsorbs to the silanol group by an electrostatic interaction may be used as the hydrophilic polymer. The silanol group is positively charged or negatively charged depending on the pH conditions to be used, and hence the hydrophilic polymer may also be caused to ionically adsorb thereto. In this case, the hydrophilic polymer that reacts with a silanol group may have a carboxy group or an amine-based ionic functional group. Specifically, for example, polyethylenimine may be used.

The hydrophilic polymer that reacts with a silanol group in one embodiment of the present invention preferably has such a molecular weight and such a concentration that the hydrophilic polymer is completely dissolved in the diluent and does not affect the viscosity of the diluent. Specifically, the weight-average molecular weight of the hydrophilic polymer is preferably 100 or more and 100,000 or less. The concentration of the hydrophilic polymer with respect to the reaction liquid in the analysis step may be set to 0.001 mass% or more and 2.0 mass% or less, and is preferably 0.03 mass% or more and 0.3 mass% or less.

(Target Substance)

Examples of the target substance may include an antigen, an antibody, a low-molecular-weight compound, various receptors, an enzyme, a substrate, a nucleic acid, a cytokine, a hormone, a neurotransmitter, a transmitter, and a membrane protein. Examples of the antigen include an allergen, a bacterium, a virus, a cell, a cell membrane constituent, a cancer marker, various disease markers, an antibody, a blood-derived substance, a food-derived substance, a natural product-derived substance, and any low-molecular-weight compound. Examples of the nucleic acid include DNA, RNA, or cDNA derived from a bacterium, a virus, or a cell, a part or fragment thereof, a synthetic nucleic acid, a primer, and a probe. Examples of the low-molecular-weight compound include a cytokine, a hormone, a neurotransmitter, a transmitter, a membrane protein, and a receptor therefor.

(Reagent)

The analysis method, the analysis apparatus, and the analysis kit according to one embodiment of the present invention may be used for a specimen test or *in vitro* diagnosis. The reagent for such use includes the luminescent reagent having a hydrophilic surface, and may include a dispersion medium for dispersing the luminescent reagent. The amount of the luminescent reagent to be incorporated into the reagent is preferably 0.000001 mass% or more and 20 mass% or less, more preferably 0.0001 mass% or more and 1 mass% or less. The reagent may include, in addition to the luminescent reagent, a third substance, such as an additive or a blocking agent, to the extent that the object of the present invention can be achieved. The reagent may include a combination of two or more kinds of third substances, such as an additive and a blocking agent. Examples of the dispersion medium include various buffer solutions, such as a phosphate buffer solution, a glycine buffer solution, a Good's buffer solution, a Tris buffer solution, and an ammonia buffer solution, but the dispersion medium is not limited thereto.

The reagent may include a ligand, and when the reagent is used for the detection of an antigen or an antibody in a specimen, an antibody or an antigen may be used as the ligand.

(Luminescent Reagent)

The luminescent reagent in one embodiment of the present invention has a hydrophilic surface, and includes the luminescent particle. In one embodiment of the present invention, the luminescent reagent is a reagent that produces luminescence, in particular, a reagent that is excited to emit light when irradiated with light, and excludes a reagent based on luminescence produced through a chemical reaction like luminol. The luminescence encompasses phosphorescence and fluorescence, but is preferably phosphorescence. In one embodiment of the present invention, the luminescent particle more preferably contains a europium complex. In addition, the luminescent reagent preferably includes a ligand specific to the target substance. That is, the luminescent reagent preferably includes a ligand that binds to the target substance. The incorporation of the ligand enables the luminescent reagent to detect/quantify the target substance based on polarization anisotropy. In one embodiment of the present invention, the "ligand" refers to a compound that specifically binds to a particular target substance.

Any compound that shows an affinity for a particular substance may be used as the ligand. Examples of the ligand and the target substance, or a combination of the target substance and the ligand may include the following. That is, the examples may include: an antigen and an antibody; a low-molecular-weight compound and a receptor therefor; an enzyme and a substrate; and nucleic acids complementary to each other. Further, the examples may include an antibody and any of the following substances specific thereto: an allergen, a bacterium, a virus, a cell, a cell membrane constituent, a cancer marker, various disease markers, an antibody, a blood-derived substance, a food-derived substance, a natural product-derived substance, and any low-molecular-weight compound. Further, the examples may include a receptor and any of the following substances specific thereto: a low-molecular-weight compound, a cytokine, a hormone, a neurotransmitter, a transmitter, and a membrane protein. Further, the examples may include DNA, RNA, or cDNA derived from a bacterium, a virus, or a cell, a part or fragment thereof, a synthetic nucleic acid, a primer, or a probe, and a nucleic acid having complementarity thereto. Other than the foregoing, any combination known to have an affinity is used as the combination of the target substance and the ligand. A typical example of the ligand is any one of an antibody, an antigen, or a nucleic acid. In addition, it is particularly preferred that the ligand be an antibody, and the target substance be an antigen.

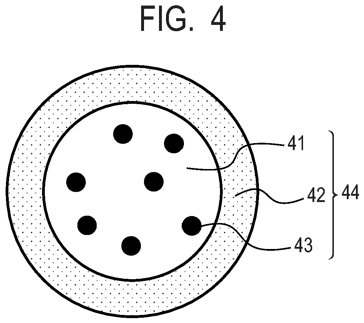

FIG. 4 is a schematic view for illustrating the luminescent reagent to be used in one embodiment of the present invention. A luminescent reagent 44 to be used in one embodiment of the present invention includes, for example, a particle substrate 41 serving as a luminescent particle containing europium complexes 43 as luminescent molecules. Further, the luminescent reagent 44 has a hydrophilic layer 42 that covers the surface thereof. The luminescent reagent to be used in one embodiment of the present invention can emit phosphorescence having a long lifetime by virtue of containing the europium complex. In the luminescent reagent to be used in one embodiment of the present invention, an average particle diameter that is the average of particle diameters of the luminescent reagent including the hydrophilic layer is preferably 25 nm or more and 500 nm or less, more preferably 50 nm or more and 300 nm or less. When the average particle diameter is more than 500 nm, the R (R0) before aggregation is increased, with the result that a difference between the R0 and the R after an aggregation reaction is reduced. In addition, when the average particle diameter is less than 25 nm, a change between sizes before and after the aggregation is reduced, with the result that it becomes difficult to grasp a change in R, for example, in measurement based on polarization anisotropy using phosphorescence.

The particle diameter of the luminescent reagent may be determined by a dynamic light scattering method. When particles dispersed in a solution are irradiated with laser light and the resultant scattered light is observed with a photon detector, an intensity distribution due to interference of the scattered light is constantly fluctuating because the particles are constantly shifting their positions by Brownian motion. The dynamic light scattering method is a measurement method for observing the state of the Brownian motion as a fluctuation in scattered light intensity. The fluctuation of scattered light with respect to time is expressed as an autocorrelation function, and a translational diffusion coefficient is determined. A Stokes diameter is determined from the determined diffusion coefficient, and the size of each of the particles dispersed in the solution can be derived.

The luminescent reagent preferably has nothing provided on the surface of the luminescent particle from the viewpoint of keeping the uniformity and monodispersity of the particles. However, the luminescent reagent to be used in one embodiment of the present invention has a hydrophilic surface, and preferably has the hydrophilic layer on the surface of the luminescent particle so that nonspecific adsorption of a substance except the target substance to the luminescent reagent is suppressed.

A method involving supporting bovine serum albumin (BSA) on the surface of the particle is widely used as a technique for keeping hydrophilicity, but this method may cause a variation between lots. Accordingly, the luminescent reagent preferably includes a hydrophilic layer containing a hydrophilic polymer for a hydrophilic layer. The hydrophilic layer is described later.

In general, in analysis for calculating the concentration of the target substance with high sensitivity, in particular, analysis based on polarization anisotropy, measurement is performed under the state in which the amounts of the target substance and the reagent that reacts with the target substance are small. In this case, the concentration of the luminescent reagent in the reaction liquid is preferably 0.000001 mass% or more and 1 mass% or less, more preferably 0.00001 mass% or more and 0.001 mass% or less.

By reducing the particle size distribution of the luminescent reagent and introducing the europium complex as the luminescent molecule, for example, a change in value (R) for polarization anisotropy is easily grasped even when the dispersion state of the particles in the liquid undergoes a slight change. Specifically, even in a case in which the concentration of the target substance in the reaction liquid is from about several picograms to about several nanograms per mL, when the luminescent reagent aggregates via the target substance, a change in rotational Brownian motion of the luminescent reagent can be grasped, for example, as a change in value (R) for polarization anisotropy.

The "polarized luminescence" refers to the following phenomenon: when a luminescent material having anisotropy in transition moment (transition dipole moment) uses polarized light along its transition moment as excitation light, its luminescence is also polarized light along the transition moment. The europium complex shows fluorescent luminescence based on energy transfer from the ligand to the central metal ion, and hence the transition moment is complicated, but red luminescence around 610 nm, which is derived from electronic transition from the lowest excited state 5D0 to 7F2, is emitted as polarized light.

The principle of the polarization anisotropy is the measurement of a shift in transition moment due to the rotational motion of the luminescent material during the occurrence of polarized luminescence. The rotational motion of the luminescent material may be represented by the equation (3):

$$Q=3V\eta/kT \cdots (3)$$

where Q represents the rotational relaxation time of the material, V represents the volume of the material, $\eta$ represents the viscosity of a solvent, "k" represents the Boltzmann constant, and T represents an absolute temperature.

The rotational relaxation time of the material is a time period required for a molecule to rotate by an angle 0 (68.5°) at which $\cos\theta=1/e$.

It is found from the equation (3) that the rotational relaxation time of the luminescent material is proportional to the volume of the material, that is, when the luminescent material has a particulate shape, the cube of the particle diameter. Meanwhile, a relationship between the emission lifetime and degree of polarization, which is a value for polarization anisotropy, of the luminescent material may be represented by the equation (4):

$$p0/p=1+A(\tau/Q) \cdots (4)$$

where p0 represents a degree of polarization at a time when the material is stationary (Q=∞), "p" represents the degree of polarization, A is a constant, $\tau$ represents the emission lifetime of the material, and Q represents the rotational relaxation time.

It is found from the equation (3) and the equation (4) that the degree of polarization is influenced by the emission lifetime of the luminescent material and the rotational relaxation time, that is, the volume (particle diameter) of the luminescent material, in other words, is influenced by the balance between the particle diameter and emission lifetime of the luminescent material.

When the degree of polarization of the luminescent material represented by the equation (4) is determined experimentally, it is appropriate that polarized light be allowed to enter the luminescent material, and luminescence be detected in a 90° direction with respect to the traveling direction and vibration direction of excitation light. In this case, it is appropriate that the detected light be detected by being divided into polarized light components in parallel and perpendicular directions with respect to the polarized light that is the incident light, and polarization anisotropy represented by, for example, the equation (5) be set as the value for polarization anisotropy:

$$r(t)=(I_{\parallel}(t)-GI_{\perp}(t))/(I_{\parallel}(t)+2GI_{\perp}(t)) \cdots (5)$$

where r(t) represents polarization anisotropy at a time "t", $I_{\parallel}(t)$ represents the luminescence intensity of a luminescence component parallel to the excitation light at the time "t", $I_{\perp}(t)$ represents the luminescence intensity of a luminescence component perpendicular to the excitation light at the time "t", and G represents a correction value, that is, the ratio of $I_{\perp}/I_{\parallel}$ measured with excitation light having a vibration direction different by 90° from that of the excitation light used for sample measurement.

That is, when the particle size and the emission lifetime fall within appropriate ranges, a change in size of the luminescent material due to, for example, a reaction with the target substance can be sensitively read as a change in polarization anisotropy. That is, the r(t) of the unaggregated luminescent material is observed to be low, and the r(t) of the aggregated luminescent material is observed to be high. This is the principle of polarization anisotropy.

The value for polarization anisotropy may be corrected with G and 2G, or may be a value obtained by removing G and 2G.

(Luminescent Particle)

The overall shape of the luminescent reagent to be used in one embodiment of the present invention and the shape of the luminescent particle (particle substrate 41 of FIG. 4) are not particularly limited. The luminescent particle is not particularly specified as long as a material thereof is capable of stably incorporating the europium complex, but is preferably a polymer containing a styrene unit and an organic silane unit. In particular, for example, a polymer obtained by polymerizing a composition containing styrene as a main component and a radically polymerizable organic silane is preferred. When the composition contains styrene as the main component, particles having an extremely uniform particle size distribution can be produced by an emulsion polymerization method to be described later. In addition, when a polymer containing an organic silane unit is adopted, a silanol group (Si-OH) is produced in the polymer in a water solvent, and the silanol groups form a siloxane bond (Si-O-Si) with each other on the luminescent particle surface, via which the hydrophilic layer to be described later or a ligand can be provided. The luminescent reagent preferably has a ligand-bonding functional group capable of bonding a ligand to the outside of the luminescent particle.

(Hydrophilic Layer)

The hydrophilic layer (hydrophilic layer 42 of FIG. 4) can be formed by incorporating a hydrophilic polymer for a hydrophilic layer or a hydrophilic molecule for a hydrophilic layer on the outside of the luminescent particle. The hydrophilic polymer for a hydrophilic layer or the hydrophilic molecule for a hydrophilic layer is a polymer or molecule containing a hydrophilic group, and specific examples of the hydrophilic group include molecules or polymers each having a hydroxy group, an ether, pyrrolidone, or a betaine structure. Specific examples of the hydrophilic polymer for a hydrophilic layer include polyethylene glycol, polyvinylpyrrolidone, a polymer of sulfobetaine, a polymer of phosphobetaine, and polyglycidyl methacrylate whose molecule has an end modified with a hydroxy group by ring-opening a glycidyl group. Those hydrophilic polymers may each be used as a main component of the hydrophilic layer. Alternatively, the hydrophilic layer may be formed by directly providing a single molecule having a hydrophilic group on the surface of the luminescent particle through use of a silane coupling agent or the like. The thickness of the hydrophilic layer is not limited, but does not need to be set to be large beyond a thickness with which hydrophilicity can be exhibited. When the hydrophilic layer is excessively thick, the hydrophilic layer may become hydrogel-like and may be hydrated by the influence of ions in the solvent, to thereby make its thickness unstable. The thickness of the hydrophilic layer is suitably 1 nm or more and 15 nm or less.

(Europium Complex)

The luminescent reagent to be used in one embodiment of the present invention may contain the europium complex (europium complex 43 of FIG. 4) as a luminescent dye. In the luminescent reagent in one embodiment of the present invention, the luminescent particle preferably contains the europium complex. The europium complex has a feature in that the wavelength and intensity of its luminescence are hardly influenced by the surroundings, and hence the luminescence has a long lifetime. The europium complex includes a europium element and a ligand. In consideration of the emission lifetime, a visible emission wavelength region, and the like, the luminescent dye is preferably a europium complex. Europium generally has an emission lifetime of from 0.1 ms to 1.0 ms. The emission lifetime and the rotational relaxation time obtained from the equation (3) need to be appropriately adjusted. In the case of europium in a water dispersion, when the diameter of the luminescent reagent is from about 50 nm to about 300 nm, for example, the value (R) for polarization anisotropy significantly changes before and after aggregation.

At least one of the constituent ligands of the europium complex is a ligand having a light-collecting function. The "light-collecting function" refers to an action of being excited at a particular wavelength to excite the central metal of the complex through energy transfer. In addition, it is preferred that the constituent ligands of the europium complex include a ligand such as a β-diketone to prevent coordination of a water molecule. The ligand such as the β-diketone coordinated to a europium ion suppresses a deactivation process due to the transfer of energy to a solvent molecule or the like to provide strong fluorescent luminescence.

The europium complex may be a polynuclear complex.

In addition, specific examples of the europium complex include [tris(2-thenoyltrifluoroacetone)(bis(triphenylphosphineoxide))europium(III)], [tris(2-thenoyltrifluoroacetone)(triphenylphosphineoxide)(dibenzylsulfoxide)europium(III)], and [tris(2-thenoyltrifluoroacetone)(phenanthroline)europium(III)].

At the time of a state in which the Brownian rotational motion of the europium complex can be regarded as stationary in a medium, the "r" represented by the equation (1) is preferably 0.08 or more. The state in which the Brownian rotational motion can be regarded as stationary refers to a state in which the rotational relaxation time of the particle is sufficiently longer than the emission lifetime of the europium complex.

The europium complex is preferably incorporated in as large an amount as possible into the luminescent particle because a luminescence intensity per particle becomes stronger. Meanwhile, when the molecules of the europium complex aggregate in the luminescent particle, an interaction between ligands influences the excitation efficiency of the europium complex and the like to make it difficult to measure the R or the like while keeping reproducibility. Whether the europium complex shows non-aggregated luminescence behavior in the luminescent particle may be judged from an excitation spectrum.

Particles having strong luminescence not only enable high-sensitivity measurement, but also enable an increase in biochemical reaction rate because luminescence is kept even when their particle diameters are reduced. As the particle diameters become smaller, the diffusion coefficient of Brownian motion in the liquid becomes larger, and hence the reaction can be detected in a shorter time period.

When a liquid having such particles dispersed therein is used in analysis in one embodiment of the present invention, for example, a change in value (R) for polarization anisotropy can be detected with high sensitivity in response to the aggregation/dispersion behavior of the particles. A dispersion obtained by dispersing such particles in a water solvent can be utilized, for example, as a high-sensitivity reagent using polarization anisotropy. A buffer solution may be used as the water solvent. In addition, a surfactant, a preservative, a sensitizer, or the like may be added into the water solvent in order to enhance the stability of the liquid having the particles dispersed therein.

(Method of producing Luminescent Reagent)

Next, an example of a method of producing the luminescent reagent to be used in one embodiment of the present invention is described.

The method of producing the luminescent reagent may include a step (first step) of mixing radically polymerizable monomers including styrene and a radically polymerizable organic silane, a radical polymerization initiator, a polarized luminescent europium complex, and a hydrophilic polymer for a hydrophilic layer with an aqueous medium to prepare an emulsion.

Further, the method of producing the luminescent reagent may include a step (second step) of heating the emulsion to polymerize the radically polymerizable monomers.

The method of producing the luminescent reagent may include a step (third step) of providing a ligand-bonding functional group to be described later on the surface of the luminescent reagent. Herein, the ligand-bonding functional group refers to a functional group that can bond a ligand. Specifically, a carboxy group, an amino group, a thiol group, an epoxy group, a maleimide group, a succinimidyl group, or an alkoxysilyl group (silicon alkoxide structure) may be used.

(Radically Polymerizable Monomers)

The production of the luminescent reagent is performed by polymerizing the radically polymerizable monomers, and the radically polymerizable monomers include at least styrene and the radically polymerizable organic silane. The radically polymerizable monomers may further include a monomer selected from the group consisting of an acrylate-based monomer; and a methacrylate-based monomer. Examples of the monomers may include butadiene, vinyl acetate, vinyl chloride, acrylonitrile, methyl methacrylate, methacrylonitrile, methyl acrylate, and mixtures thereof. That is, one kind or a plurality of kinds of those monomers may be used in addition to styrene and the radically polymerizable organic silane. In addition, a monomer having two or more double bonds per molecule such as divinylbenzene may be used as a crosslinking agent.

The incorporation of the radically polymerizable organic silane in the radically polymerizable monomers provides a siloxane bond to the luminescent particle. Examples of the radically polymerizable organic silane may include vinyltrimethoxysilane, vinyltriethoxysilane, p-styryltrimethoxysilane, 3-methacryloxypropylmethyldimethoxysilane, 3-methacryloxypropyltrimethoxysilane, 3-methacryloxypropylmethyldiethoxysilane, 3-methacryloxypropyltriethoxysilane, 3-acryloxypropyltrimethoxysilane, and combinations thereof. The use of the radically polymerizable organic silane serves to form a backbone of an inorganic oxide in the luminescent particle to improve the physical and chemical stability of the luminescent reagent. Further, the use of the radically polymerizable organic silane enhances an affinity between the luminescent particle and each of the hydrophilic layer and the ligand-bonding functional group.

Further, the incorporation of the radically polymerizable organic silane in the radically polymerizable monomers provides a silanol group to the surface of the luminescent particle. The silanol group and the hydrophilic polymer for a hydrophilic layer, such as PVP, form a hydrogen bond. Thus, the hydrophilic polymer for a hydrophilic layer, such as PVP, more strongly adsorbs to the surface of the luminescent particle, thereby allowing formation of a hydrophilic layer.

(Radical Polymerization Initiator)

A wide range of compounds selected from, for example, azo compounds and organic peroxides may each be used as the radical polymerization initiator. Specific examples thereof may include 2,2'-azobis(isobutyronitrile), 2,2'-azobis(2,4-dimethylvaleronitrile), 2,2'-azobis(2-methylbutyronitrile), 4,4'-azobis(4-cyanovaleric acid), 2,2'-azobis(2-methylpropionamidine) dihydrochloride, dimethyl 2,2'-azobis(2-methylpropionate), tert-butyl hydroperoxide, benzoyl peroxide, ammonium persulfate (APS), sodium persulfate (NPS), and potassium persulfate (KPS).

(Hydrophilic Polymer for Hydrophilic Layer)

The luminescent reagent may include the hydrophilic polymer for a hydrophilic layer as the hydrophilic layer. The hydrophilic polymer for a hydrophilic layer is preferably capable of suppressing nonspecific adsorption. Examples of the hydrophilic polymer for a hydrophilic layer include hydrophilic polymers for a hydrophilic layer each containing a unit having an ether, a betaine, or a pyrrolidone ring. It is preferred that the hydrophilic layer be included in the synthesized luminescent reagent, and be mainly present on the particle surface on the outside of the luminescent particle. For example, when the PVP is loaded at the time of the synthesis of the luminescent reagent, a nonspecific adsorption-suppressing ability and a ligand-bonding ability can be simultaneously imparted to the luminescent reagent. The PVP to be loaded at the time of the synthesis has higher hydrophilicity than that of each of the radically polymerizable monomers, and hence is present at an interface between the solvent and the luminescent particle that is being polymerized at the time of the synthesis. The luminescent particle can adsorb the PVP or the like on the outside thereof by involving part of the PVP at the time of the polymerization, or by physical/chemical adsorption such as an interaction between a pyrrolidone ring and styrene (radically polymerizable monomer).

The weight-average molecular weight of the PVP is preferably 10,000 or more and 100,000 or less, more preferably 40,000 or more and 70,000 or less. When the weight-average molecular weight is less than 10,000, the hydrophilicity of the surface of the luminescent reagent is weak, and hence nonspecific adsorption is liable to occur. When the weight-average molecular weight is more than 100,000, the hydrophilic layer becomes so thick as to gel, thereby becoming difficult to handle.

In addition to the PVP, another hydrophilic polymer for a hydrophilic layer may be added as a protective colloid at the time of the synthesis of the luminescent reagent.

In addition, the luminescent reagent preferably satisfies A2-A1≤0.1.

A1 and A2 are defined as described below. That is, with regard to a mixture obtained by adding 30 $\mu$L of a 0.1 mass% dispersion of the luminescent reagent to 60 $\mu$L of a buffer solution mixed with 16 $\mu$L of human serum diluted 15-fold, the absorbance of the mixture immediately after the addition is represented by A1, and the absorbance of the mixture after being left to stand at 37°C for 5 minutes after the addition is represented by A2. The absorbances are measured at an optical path of 10 mm and a wavelength of 572 nm.

A particle showing an A2-A1 of 0.1 or less has little nonspecific adsorption of impurities in serum, and is hence preferred.

(Aqueous Medium)

The aqueous medium (aqueous solution) to be used for the above-mentioned method of producing the luminescent reagent preferably contains water at 80 mass% or more and 100 mass% or less in the medium. The aqueous medium is preferably water or a water-soluble organic solvent, and examples thereof include solutions each obtained by mixing water with methanol, ethanol, isopropyl alcohol, or acetone. When an organic solvent other than water is incorporated at more than 20 mass%, dissolution of the polymerizable monomers may occur at the time of the production of the luminescent reagent.

In addition, the aqueous medium preferably has its pH adjusted to 6 or more and 9 or less in advance. When the pH has a value of less than 6 or more than 9, an alkoxy group or silanol group of the radically polymerizable organic silane may undergo condensation polymerization or a reaction with another functional group before the formation of the polymer, leading to aggregation of the particles to be obtained. In this example, the alkoxide is not intentionally subjected, before the polymerization, to condensation polymerization.

The above-mentioned pH is preferably adjusted with a pH buffer, but may be adjusted with an acid or a base.

Other than the foregoing, a surfactant, an antifoaming agent, a salt, a thickener, or the like may be used by being added at a ratio of 10 mass% or less with respect to the aqueous medium.

In the production of the luminescent reagent, it is preferred that, first, the PVP be dissolved in the aqueous medium whose pH has been adjusted to from 6 to 9. The content of the PVP is preferably 0.01 mass% or more and 10 mass% or less, more preferably 0.03 mass% or more and 5 mass% or less with respect to the aqueous medium. When the content is less than 0.01 mass%, the amount of adsorption onto the luminescent particle is small, and the effect thereof is not expressed. In addition, when the content is more than 10 mass%, the viscosity of the aqueous medium may be increased to preclude sufficient stirring.

Subsequently, the radically polymerizable monomers including the styrene (A) and the radically polymerizable organic silane (B) are added into the above-mentioned aqueous medium to prepare an emulsion. A weight ratio between the styrene (A) and the radically polymerizable organic silane (B) is from 6:4 to 100:1. Further, the prepared emulsion is mixed with the europium complex. At this time, when the solubility of the europium complex is low, a water-insoluble organic solvent may be added. A weight ratio between the europium complex and the radically polymerizable monomers is from 1:1,000 to 1:10.

When the weight ratio of the styrene (A) to the radically polymerizable organic silane (B) is less than 6/4, the specific gravity of the particles as a whole may be increased, resulting in remarkable sedimentation of the particles. In addition, in order to increase adhesiveness between the PVP and the luminescent particle, it is preferred that the weight ratio of the styrene (A) to the radically polymerizable organic silane (B) be set to 100/1 or less.

A weight ratio between the weight of the aqueous medium and the total amount of the radically polymerizable monomers is preferably from 5:5 to 9.5:0.5. When the weight ratio of the weight of the aqueous medium to the total amount of the radically polymerizable monomers is less than 5/5, remarkable aggregation of the particles to be produced may occur. In addition, when the weight ratio of the weight of the aqueous medium to the total amount of the radically polymerizable monomers is more than 9.5/0.5, although there is no problem with the production of the particles, the production amount thereof may be reduced.

The radical polymerization initiator is used by being dissolved in water, a buffer, or the like. The amount of the radical polymerization initiator may be set to 0.5 mass% or more and 10 mass% or less in the emulsion with respect to the total amount of the styrene (A) and the radically polymerizable organic silane (B).

In the above-mentioned step of heating the emulsion, it is only required that the entire emulsion be uniformly heated. A heating temperature may be arbitrarily set between 50°C and 80°C, and a heating time may be arbitrarily set between 2 hours and 24 hours. Through the heating of the emulsion, the radically polymerizable monomers are polymerized.

The luminescent reagent may have a ligand-bonding functional group on the surface thereof. The ligand-bonding functional group may be provided to the hydrophilic layer or the luminescent particle of the luminescent reagent. The ligand-bonding functional group is not particularly limited as long as the functional group can bond an antibody, an antigen, an enzyme, or the like. However, for example, the functional group may be a carboxy group, an amino group, a thiol group, an epoxy group, a maleimide group, a succinimidyl group, or a silicon alkoxide group, or contain a group having any of those functional groups. For example, a silane coupling agent having the ligand-bonding functional group and the synthesized particle may be mixed to provide the functional group on the particle surface. Specifically, an aqueous solution of a silane coupling agent having a carboxy group may be prepared and mixed with a dispersion of the synthesized particle to provide the carboxy group to the particle surface. At this time, a dispersant such as Tween 20 may be added to the reaction solution. A reaction temperature may be arbitrarily set between 0°C and 80°C, and a reaction time may be arbitrarily set between 1 hour and 24 hours. In order to suppress an abrupt condensation reaction of the silane coupling agent, it is suitable that the temperature be set to be equal to or lower than a room temperature of about 25°C, and the reaction time be set to from about 3 hours to about 14 hours.

Depending on the ligand-bonding functional group, the reaction with the particle surface may be promoted by adding an acid or alkali catalyst.

The luminescent reagent can be utilized as a particle for a specimen test by bonding a ligand such as any of various antibodies thereto. An optimal technique for bonding an antibody of interest or the like through utilization of a functional group present on the hydrophilic layer or the luminescent particle may be selected.

(Introduction of Ligand)

A hitherto known method may be applied to a chemical reaction for chemically bonding the ligand-bonding functional group and the ligand to the extent that the object of the present invention can be achieved. In addition, when the ligand is amide-bonded, a catalyst such as 1-[3-(dimethylaminopropyl)-3-ethylcarbodiimide] may be appropriately used.

The luminescent reagent to be used in an embodiment of the present invention may be applied to a latex immunoagglutination measurement method utilized widely in the fields of clinical tests, biochemical research, and the like, but is preferably used in the analysis method based on polarization anisotropy.

(Analysis Apparatus)

The present invention provides the following analysis apparatus as one embodiment.

The analysis apparatus according to one embodiment of the present invention is an analysis apparatus for determining at least any one of presence or absence of a target substance; and a concentration of the target substance, the analysis apparatus including: a glass vessel to be used for accommodating a reaction liquid containing: a sample containing the target substance; and a reagent that reacts with the target substance; a unit configured to load the sample containing the target substance into the glass vessel; a unit configured to load a hydrophilic polymer that reacts with a silanol group of the glass vessel into the glass vessel; a unit configured to load the reagent into the glass vessel after or simultaneously with the loading of the hydrophilic polymer; an irradiation unit configured to irradiate, as irradiation light, the glass vessel with light that is linearly polarized light; a measurement unit configured to measure fluorescence intensities of fluorescence of two polarized light components in a parallel direction and an orthogonal direction with respect to the linearly polarized light; and a processing unit configured to process results obtained by the measurement unit, to thereby determine at least any one of: the presence or absence of the target substance; and the concentration of the target substance in the reaction liquid, wherein the reagent includes a luminescent reagent having a hydrophilic surface, and the luminescent reagent includes a luminescent particle.

In addition, the analysis apparatus according to one embodiment of the present invention is an analysis apparatus for determining at least any one of: the presence or absence of a target substance; and the concentration of the target substance, the analysis apparatus including: a glass vessel to be used for accommodating a reaction liquid containing: a sample containing the target substance; and a reagent that reacts with the target substance; a unit configured to load the sample containing the target substance into the glass vessel; a unit configured to load a hydrophilic polymer that reacts with a silanol group of the glass vessel into the glass vessel; a unit configured to load the reagent into the glass vessel; an irradiation unit configured to irradiate, as irradiation light, the glass vessel with light that is linearly polarized light; a measurement unit configured to measure fluorescence intensities of fluorescence of two polarized light components in a parallel direction and an orthogonal direction with respect to the linearly polarized light; and a processing unit configured to process results obtained by the measurement unit, to thereby determine at least any one of the presence or absence of the target substance; and the concentration of the target substance in the reaction liquid, wherein the processing unit is configured to perform control so that the reagent is loaded after or simultaneously with the loading of the hydrophilic polymer into the glass vessel.

[Example of Configuration of Analysis Apparatus]

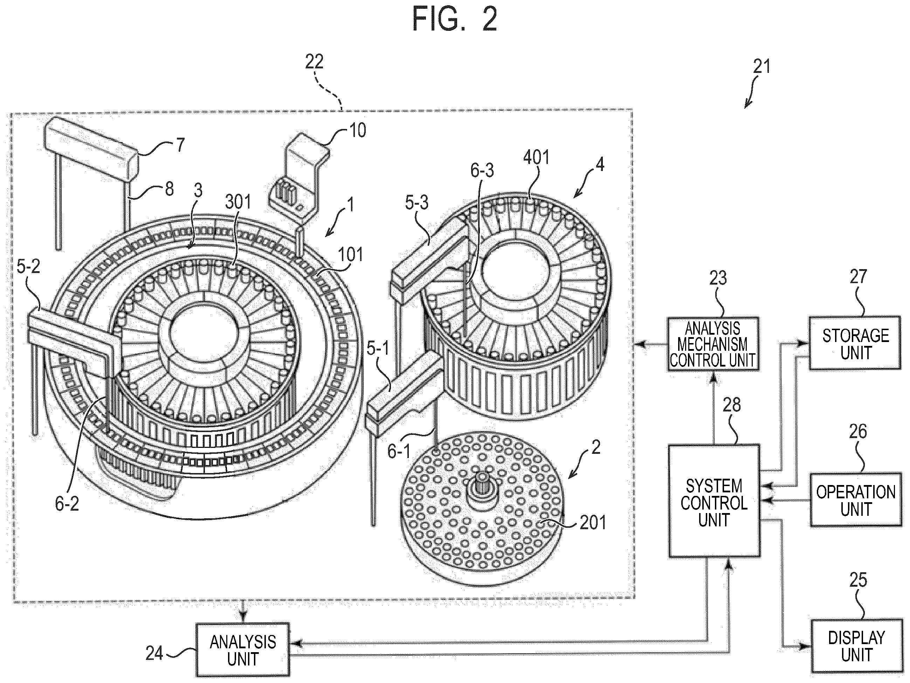

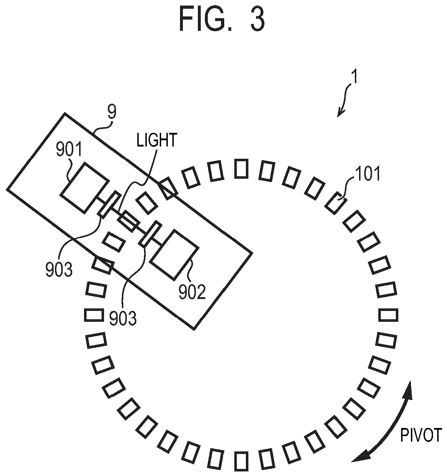

An example of the configuration of the analysis apparatus is described with reference to FIG. 2 and FIG. 3. In the analysis apparatus, the presence or absence of the target substance, or the concentration of the target substance can be determined by stirring the sample, such as blood or urine, and the reagent in the glass vessel, which is a vessel having a square tube shape, to cause a reaction therebetween, and performing, for example, measurement based on polarization anisotropy. As illustrated in FIG. 2, an analysis apparatus 21 includes an analysis mechanism 22, an analysis mechanism control unit 23, an analysis unit 24, a display unit 25, an operation unit 26, a storage unit 27, and a system control unit 28.

The analysis mechanism 22 is operated in accordance with control by the analysis mechanism control unit 23. The analysis mechanism 22 is arranged in a casing in the analysis apparatus 21. For example, as illustrated in FIG. 2 and FIG. 3, the analysis mechanism 22 has mounted thereto a reaction disc 1, a sample disc 2, a polymer accommodation tank 3, a reagent tank 4, a sample arm 5-1, a sample probe 6-1, a polymer loading arm 5-2, a polymer loading probe 6-2, a reagent arm 5-3, a reagent probe 6-3, a stirring arm 7, a stirring bar 8, a photometric mechanism 9, and a washing mechanism 10.

As illustrated in FIG. 2, the reaction disc 1 holds a plurality of glass vessels 101 arranged on its circumference. The reaction disc 1 alternately repeats rotation and stop at predetermined time intervals. The sample disc 2 is arranged in the vicinity of the reaction disc 1. The sample disc 2 holds a sample vessel 201 in which the sample containing the target substance is accommodated. The sample disc 2 rotates so that the sample vessel 201 in which the sample containing the target substance is accommodated is arranged at a sample suction position. The polymer accommodation tank 3 holds a plurality of polymer accommodation vessels 301 in each of which the hydrophilic polymer is accommodated. The polymer accommodation tank 3 rotates so that the polymer accommodation vessel 301 in which the hydrophilic polymer to be dispensed is accommodated is arranged at a polymer suction position. The reagent tank 4 is arranged in the vicinity of the reaction disc 1. The reagent tank 4 holds a plurality of reagent vessels 401 in each of which the reagent is accommodated. The reagent tank 4 rotates so that the reagent vessel 401 in which the reagent to be dispensed is accommodated is arranged at a reagent suction position.

The sample arm 5-1 is arranged between the reaction disc 1 and the sample disc 2.

The sample probe 6-1 is attached to the tip of the sample arm 5-1. The sample arm 5-1 supports the sample probe 6-1 in a vertically movable manner. In addition, the sample arm 5-1 supports the sample probe 6-1 in a pivotable manner along a pivot track in an ark shape. The pivot track of the sample probe 6-1 passes the sample suction position above the sample disc 2 and a sample discharge position above the reaction disc 1. The sample probe 6-1 suctions the sample from the sample vessel 201 arranged at the sample suction position above the sample disc 2, and discharges the sample into the glass vessel 101 arranged at the sample discharge position above the reaction disc 1.

The polymer loading arm 5-2 is arranged in the vicinity of the outer periphery of the reaction disc 1. The polymer loading probe 6-2 is attached to the tip of the polymer loading arm 5-2. The polymer loading arm 5-2 supports the polymer loading probe 6-2 in a vertically movable manner. In addition, the polymer loading arm 5-2 supports the polymer loading probe 6-2 in a pivotable manner along a pivot track in an ark shape. The pivot track of the polymer loading probe 6-2 passes the polymer suction position above the polymer accommodation tank 3 and a polymer discharge position above the reaction disc 1. The polymer loading probe 6-2 suctions the hydrophilic polymer from the polymer accommodation vessel 301 arranged at the polymer suction position above the polymer accommodation tank 3, and discharges the hydrophilic polymer into the glass vessel 101 arranged at the polymer discharge position above the reaction disc 1.

The reagent arm 5-3 is arranged between the reaction disc 1 and the reagent tank 4. The reagent probe 6-3 is attached to the tip of the reagent arm 5-3. The reagent arm 5-3 supports the reagent probe 6-3 in a vertically movable manner. In addition, the reagent arm 5-3 supports the reagent probe 6-3 in a pivotable manner along a pivot track in an ark shape. The pivot track of the reagent probe 6-3 passes the reagent suction position above the reagent tank 4 and a reagent discharge position above the reaction disc 1. The reagent probe 6-3 suctions the reagent from the reagent vessel 401 arranged at the reagent suction position above the reagent tank 4, and discharges the reagent into the glass vessel 101 arranged at the reagent discharge position above the reaction disc 1.

The stirring arm 7 is arranged in the vicinity of the outer periphery of the reaction disc 1. The stirring bar 8 is attached to the tip of the stirring arm 7. The stirring arm 7 supports the stirring bar 8 in a vertically movable manner. In addition, the stirring arm 7 supports the stirring bar 8 in a pivotable manner along a pivot track in an ark shape. The stirring bar 8 stirs a liquid containing the sample and the reagent in the glass vessel 101 arranged at a stirring position above the reaction disc 1.

The glass vessel 101 is a vessel for accommodating the reaction liquid. It is desired that, out of the surfaces of the glass vessel 101, at least a light incident surface and a light exit surface be optically transparent and smooth so that optical measurement can be performed by the photometric mechanism 9.

The liquid amount of the reaction liquid changes in accordance with the amounts of the sample and the reagent, or changes in accordance with a test item. The reaction liquid is accommodated in the glass vessel 101 in a liquid amount between the minimum liquid amount and the maximum liquid amount. The maximum liquid amount is a liquid amount set for the analysis apparatus 21, and is the maximum liquid amount of the reaction liquid that can be tested. The minimum liquid amount is a liquid amount set for the analysis apparatus 21, and is the minimum liquid amount of the reaction liquid that can be tested.

As illustrated in FIG. 3, the photometric mechanism 9 is arranged in the vicinity of the reaction disc 1. The photometric mechanism 9 is operated in accordance with control by the analysis mechanism control unit 23. Specifically, the photometric mechanism 9 has a light source 901, a detector 902, and polarizers 903. The light source 901 radiates light toward the reaction liquid in the glass vessel 101 located at a photometric position in the reaction disc 1. The glass vessel 101 is pivoted by the reaction disc 1 at predetermined time intervals so that the glass vessel 101 crosses the light, which has been radiated from the light source 901 and passed through the polarizer 903, substantially at a right angle at the photometric position. The reaction liquid in the glass vessel 101 is measured by the photometric mechanism 9 every time the glass vessel 101 crosses the photometric position. The detector 902 is arranged at a position opposite to the light source 901 across the glass vessel 101 at the photometric position. The detector 902 detects light having passed through the polarizer 903 in a luminescence component from the reaction liquid in the glass vessel 101. As the polarizer 903 on a glass vessel 101 pass side (detector 902 side), two kinds of polarizers, that is, a polarizer parallel to the light polarization direction of the polarizer on an excitation light side (light source 901 side) and a polarizer perpendicular thereto are prepared. In order to prepare two kinds of the polarizers 903 on the glass vessel 101 pass side, luminescence from the sample may be divided into a plurality of directions with a splitter. Alternatively, the light polarization direction of the polarizer 903 on the glass vessel 101 pass side may be switched to enable measurement. The detector 902 produces data having measurement values corresponding to the intensities of light having been detected (hereinafter referred to as "photometric data"). The photometric data having been produced is supplied to the analysis unit 24.

For example, a halogen lamp, a light-emitting diode (LED), or a laser generator may be used as the light source 901. The light radiated from the light source 901 preferably includes light in a wavelength band in which the value for polarization anisotropy of the reaction liquid can be measured.

The detector 902 converts the intensity of light having been detected into an electrical signal. Specifically, a photomultiplier tube, a photodiode, or an arrayed photomultiplier tube or photodiode is used as the detector 902. In order to make the S/N ratio of detection satisfactory, an optical window or a concentrator may be arranged between the light source 901 and the detector 902 as required.

The washing mechanism 10 is arranged on the outer periphery of the reaction disc 1. The washing mechanism 10 is operated in accordance with control by the analysis mechanism control unit 23. Specifically, a washing nozzle and a drying nozzle are attached to the washing mechanism 10. The washing mechanism 10 washes the glass vessel 101 located at a washing position of the reaction disc 1 with the washing nozzle, and dries the glass vessel 101 with the drying nozzle.

The analysis mechanism control unit 23 operates the respective devices and mechanisms of the analysis mechanism 22 in accordance with control by the system control unit 28. The analysis unit 24 calculates the value for polarization anisotropy of the reaction liquid based on the photometric data. In addition, the analysis unit 24 quantitatively analyzes the target substance in accordance with the test item based on the value for polarization anisotropy of the reaction liquid having been calculated. The display unit 25 has a display device, such as a CRT display, a liquid crystal display, an organic EL display, or a plasma display. The display unit 25 displays analysis results obtained by the analysis unit 24. The operation unit 26 receives various commands or information input from an operator via input equipment. A pointing device, such as a mouse or a trackball, a selection device such as a switch bottom, or an input device such as a keyboard may be appropriately utilized as the input equipment. The storage unit 27 stores, for example, an operation program of the analysis apparatus 21. The system control unit 28 functions as the center of the analysis apparatus 21. The system control unit 28 reads out the operation program from the storage unit 27, and controls the respective units 23, 24, 25, and 27 in accordance with the operation program.

(Analysis Kit)

The present invention provides the following analysis kit as one embodiment.

The analysis kit according to one embodiment of the present invention is an analysis kit to be used in an analysis method for determining at least any one of: presence or absence of a target substance; and a concentration of the target substance based on polarization anisotropy, the analysis kit including: a reagent that reacts with the target substance; a glass vessel to be used for accommodating a reaction liquid containing: a sample containing the target substance; and the reagent; and a hydrophilic polymer that reacts with a silanol group of the glass vessel, wherein the reagent includes a luminescent reagent having a hydrophilic surface, and the luminescent reagent includes a luminescent particle.

[Examples]

The present invention is specifically described below by way of Examples. However, the present invention is not limited to these Examples.

(1) Production of Luminescent Reagent

Polyvinylpyrrolidone (PVP-K30: manufactured by Tokyo Chemical Industry Co., Ltd.) was dissolved in a 2-morpholinoethanesulfonic acid (MES) buffer solution (manufactured by Kishida Chemical Co., Ltd.) having a pH of 7 to prepare a solution A.

Subsequently, the following materials were mixed to prepare a mixed solution B.

·[Tris(2-thenoyltrifluoroacetone)(bis(triphenylphosphineoxide))europium(III)] (manufactured by Central Techno Corporation) serving as a europium complex
·Styrene monomer (manufactured by Kishida Chemical Co., Ltd.)
·3-Methacryloxypropyltrimethoxysilane (manufactured by Tokyo Chemical Industry Co., Ltd., hereinafter abbreviated as "MPS")

The mixed solution B was added into a four-necked flask containing the solution A, and the mixture was stirred with a mechanical stirrer set to 300 rpm. After 15 minutes of stirring under a nitrogen flow condition, the temperature of an oil bath that had been prepared was set to 70°C, and the nitrogen flow was performed for an additional 15 minutes. After the mixture had been heated and stirred, an aqueous solution having dissolved therein potassium persulfate (hereinafter abbreviated as "KPS") (manufactured by Sigma-Aldrich) was added into the reaction solution, and emulsion polymerization was performed for 20 hours. After the polymerization reaction, the resultant suspension was subjected to ultrafiltration with about 4 L of ion-exchanged water through use of an ultrafiltration membrane having a molecular weight cutoff of 100K so that the product was washed. Thus, a dispersion of a luminescent reagent was obtained.

An aliquot of the dispersion of the luminescent reagent obtained by the emulsion polymerization was taken and added to an aqueous solution having dissolved therein 1 mass% of Tween 20 (manufactured by Kishida Chemical Co., Ltd.). After 10 minutes of stirring, a silane coupling agent, X12-1135 (manufactured by Shin-Etsu Chemical Co., Ltd.), was added, and the mixture was stirred overnight. After the stirring, the dispersion was centrifuged, the supernatant was removed, and the precipitate was redispersed with pure water. The operations of centrifugation and redispersion were performed 3 or more times to wash the product. The precipitate after the washing was redispersed in pure water. Thus, a ligand-bonding functional group was introduced into the luminescent reagent. A mass ratio among the luminescent reagent, pure water, and X12-1135 loaded was set to 1:300:2.

(Production of Anti-CRP Antibody-modified Luminescent Reagent)

An aliquot of 0.25 mL of the particle dispersion at 1.2 mass% corresponding to the synthesized luminescent reagent was taken, and the solvent was replaced by 1.6 mL of a MES buffer solution having a pH of 6.0. To the particle MES buffer solution, 1-[3-(dimethylamino)propyl]-3-ethylcarbodiimide and N-hydroxysulfosuccinimide sodium were added at 0.5 mass%, and the mixture was subjected to a reaction at 25°C for 1 hour. After the reaction, the dispersion was washed with a MES buffer solution having a pH of 5.0, an anti-CRP antibody was added at 100 μg/mL, and the anti-CRP antibody was bonded to the particles at 25°C for 2 hours. After the bonding, the particles were washed with a Tris buffer solution having a pH of 8. After the reaction, the particles were washed with a phosphate buffer solution to provide an anti-CRP antibody-modified luminescent reagent having a concentration of 0.3 mass% (sometimes referred to as "affinity particles").

The bonding of the antibody to the particles was recognized by measuring the amount of a reduction in antibody concentration in the buffer solution having added thereto the antibody by BCA assay.

(Preparation of Luminescent Reagent Liquid)

The resultant luminescent reagent was diluted with a phosphate (PBS) buffer solution having a pH of 7.4 so as to have a concentration of 0.1 mg/mL to prepare a luminescent reagent liquid.

(Preparation of Diluent)

A 4-(2-hydroxyethyl)-1-piperazineethanesulfonic acid (HEPES) buffer solution and a PBS buffer solution were mixed at a ratio of 1:1 in terms of volume ratio to prepare a diluent.

(Example 1)

A liquid, which was obtained by dissolving polyvinylpyrrolidone (weight-average molecular weight: about 40,000) in the luminescent reagent liquid so as to have a concentration of 0.25 mass%, was loaded into a quartz glass cell by 2 mL, and was subjected to immersion for 72 hours. After the immersion, the luminescent reagent liquid was removed, and the glass cell was rinsed with pure water several times. After the washing, measurement was performed through use of an apparatus 1 described later.

(Example 2)

A liquid, which was obtained by dissolving polyvinylpyrrolidone (weight-average molecular weight: about 40,000) in the luminescent reagent liquid so as to have a concentration of 0.125 mass%, was loaded into a quartz glass cell by 2 mL, and was subjected to immersion for 72 hours. After the immersion, the luminescent reagent liquid was removed, and the glass cell was rinsed with pure water several times. After the washing, measurement was performed through use of the apparatus 1 described later.

(Example 3)

A liquid, which was obtained by dissolving polyvinylpyrrolidone (weight-average molecular weight: about 40,000) in the luminescent reagent liquid so as to have a concentration of 0.0625 mass%, was loaded into a quartz glass cell by 2 mL, and was subjected to immersion for 72 hours. After the immersion, the luminescent reagent liquid was removed, and the glass cell was rinsed with pure water several times. After the washing, measurement was performed through use of the apparatus 1 described later.

(Example 4)

A liquid, which was obtained by dissolving polyvinylpyrrolidone (weight-average molecular weight: about 40,000) in the luminescent reagent liquid so as to have a concentration of 0.03125 mass%, was loaded into a quartz glass cell by 2 mL, and was subjected to immersion for 72 hours. After the immersion, the luminescent reagent liquid was removed, and the glass cell was rinsed with pure water several times. After the washing, measurement was performed through use of the apparatus 1 described later.

(Comparative Example 1)

The luminescent reagent liquid was loaded into a quartz glass cell by 2 mL, and was subjected to immersion for 72 hours. After the immersion, the luminescent reagent liquid was removed, and the glass cell was rinsed with pure water several times. After the washing, measurement was performed through use of the apparatus 1 described later.

(Comparative Example 2)

After having been diluted 10-fold, the luminescent reagent liquid was loaded into a quartz glass cell by 2 mL, and was subjected to immersion for 72 hours. After the immersion, the luminescent reagent liquid was removed, and the glass cell was rinsed with pure water several times. After the washing, measurement was performed through use of the apparatus 1 described later.

(Reference Example 1)

The luminescent reagent liquid was loaded into a polymethyl methacrylate cell by 2 mL, and was subjected to immersion for 72 hours. After the immersion, the luminescent reagent liquid was removed, and the glass cell was rinsed with pure water several times. After the washing, measurement was performed through use of the apparatus 1 described later.

(Example 5)

A liquid, which was obtained by adding polyvinylpyrrolidone (weight-average molecular weight: about 40,000) to the diluent so as to have a concentration of 0.25 mass%, was loaded into a quartz glass cell by 170 $\mu$L, mixed with 10 $\mu$L of a CRP antigen liquid, and then mixed with 20 $\mu$L of the luminescent reagent liquid, and measurement was performed. The "r" in the equation (1) was measured. The investigation was performed at a concentration of a CRP antigen of from 0 pM to 200 pM. An operation in which washing of the quartz glass cell having been used and the measurement were repeated was performed 10 times. The average value of "r"s, which were obtained by performing the measurement 10 times, was determined. The amount of change in "r" with respect to the concentration of the CRP antigen corresponds to a value obtained by dividing the amount (dr) of change, which is obtained by subtracting the average value of "r"s each obtained through measurement immediately after mixing from the average value of "r"s each obtained through measurement after performing the reaction for 10 minutes, by the time (10 minutes, dt). The measurement was performed through use of an apparatus 2 described later.

(Comparative Example 3)

170 $\mu$L of the diluent and 10 $\mu$L of a CRP antigen liquid were mixed with each other, and then mixed with 20 $\mu$L of the luminescent reagent liquid, and the R was measured. The investigation was performed at a concentration of a CRP antigen of from 0 pM to 200 pM. An operation in which washing of a quartz cell having been used and the measurement were repeated was performed 10 times. The average value of "r"s, which were obtained by performing the measurement 10 times, was determined. The determination method for the amount of change in "r" is the same as in Example 5. The measurement was performed through use of the apparatus 2 described later.

(Measurement)

The shape of the resultant luminescent reagent was measured with an electron microscope (S-5500 manufactured by Hitachi High-Technologies Corporation).
The average particle diameter of the luminescent reagent was measured by using dynamic light scattering (Zetasizer Nano S manufactured by Malvern).
The concentration of a suspension having the luminescent reagent dispersed therein was measured with a gravimetric analyzer (Thermo plus TG8120 manufactured by Rigaku Corporation).
The apparatus 1 and the apparatus 2 were each used for measurement for performance evaluation. The apparatus 1 was an apparatus having such a configuration as described below, and measurement described below was performed.
An LED light source of excitation light at 340 nm was prepared, and a polarizing filter (NSPFU-30C, manufactured by Sigmakoki Co., Ltd.) and a shortpass filter (84-706, manufactured by Edmund Optics) were inserted into an optical path to set an optical system capable of irradiating a 1 cm quartz square cell. A polarizing filter (PIVISC050, manufactured by Thorlabs, Inc.) and a bandpass filter (FB610-10, manufactured by Thorlabs, Inc.) were set in a direction of 90° with respect to incident light. In order to measure luminescence as Ivv and $I_{VH}$ in two directions at the same time, two sets in which the construction of a polarizer was changed by a direction of 90° with respect to incident light were prepared. For the detection of polarized light, spectrometry was performed through use of QEPro manufactured by Ocean Optics, Inc. Temperature control was set for a sample holder so as to enable measurement at 37°C. Measurement of fluorescence was performed with the LED light source being fixed at an output of 12 mW and a cumulative time being set to 3 seconds. Based on the fluorescence spectrum, a luminescence intensity in the wavelength range of from 600 nm to 630 nm was determined.

The apparatus 2 was an apparatus having such a configuration as described below, and measurement described below was performed.

An LED light source of excitation light at 340 nm was prepared, and a polarizing filter (NSPFU-30C, manufactured by Sigmakoki Co., Ltd.) and a shortpass filter (84-706, manufactured by Edmund Optics) were inserted into an optical path to set an optical system capable of irradiating a quartz cell having an optical path length of 5 mm. For polarized luminescence emitted from a sample, the polarized light was separated into two directions by setting an excitation light cut filter (33-910, manufactured by Edmund Optics), a polarizing beamsplitter (47-127, manufactured by Edmund Optics), and a polarizer (SPF-30C-32, manufactured by Sigmakoki Co., Ltd.) in the stated order in the optical path in a straight line with the excitation light across the sample. The separated polarized luminescence (in each of the two directions) was detected with an avalanche photodiode (APD, C15522-3010SA, manufactured by Hamamatsu Photonics K.K.). Temperature control was set for a sample holder so as to enable measurement at 37°C. Measurement of signals of the polarized luminescence was performed with the LED light source being fixed at an output of 60 mW and a cumulative time being set to 8 milliseconds. The resultant signals of the polarized luminescence were measured with an oscilloscope, and were substituted into the equation (1) to determine "r". The "r" is a value (R) for polarization anisotropy. The amount of change between "r" immediately after mixing between the luminescent reagent liquid and the CRP antigen liquid and "r" after immersion for 10 minutes from the mixing was calculated.

(Performance Evaluation)

The synthesized luminescent reagent had a particle diameter of about 100 nm, and showed strong red luminescence with excitation light at 340 nm.

According to the results of the nonspecific agglutination suppression evaluation, the change in absorbance was equal to or less than the specific numerical value, and hence it was recognized that the particles were capable of suppressing nonspecific adsorption.

The results of Examples 1, 2, 3, and 4, Comparative Examples 1 and 2, and Reference Example 1 are shown in Table 1. In all Examples, the fluorescence intensities $I_{VV}$ and $I_{VH}$ in two directions were each within 100 counts, which was almost on the same level as a background. That is, it was recognized that the luminescent particles did not adhere to the wall surface of the cell even through immersion for a long time period, and were removed by the simple rinsing operation with pure water. In Comparative Examples 1 and 2, the fluorescence intensities were each beyond 10,000 counts, and definite luminescence was recognized. This indicates that the luminescent particles adhered to the wall surface through immersion for a long time period. Meanwhile, in Reference Example 1, fluorescence was not detected as in Examples, and the adhesion of the particles on the wall surface of the cell was not recognized.

In Examples 1, 2, 3, and 4, Comparative Examples 1 and 2, and Reference Example 1, when the cell after measurement of the fluorescence intensities was irradiated with UV light at 360 nm, it was visually observed that the wall surface of the cell had red luminescence in each of Comparative Examples 1 and 2. In each of Examples 1, 2, 3, and 4 and Reference Example 1, red luminescence was not observed, and the adhesion of the luminescent particles was not visually observed.

In addition, in Example 5, it was able to be recognized that the "r" derived from the equation (1) and the CRP concentration correlated to each other.

The results of the "r" in Example 5 are shown in Table 2. As shown in Table 2, it was recognized that the "r" was increased in accordance with the CRP concentration. Between 0 pM and 100 pM, it can be recognized that the CRP concentration and the change in slope (dr/dt), which is obtained by dividing the amount of change in "r" over 10 minutes by the time, particularly highly correlate to each other. It was recognized that when the CRP concentration was increased up to 200 pM, a prozone, in which a change in polarization anisotropic property was saturated, was reached, and hence the correlation was reduced. It was recognized that the correlation between the CRP concentration and the dr/dt did not significantly change over the 10-times measurement, and even the 1st measurement and the 10th measurement were able to be performed in the same manner. Further, after the 10th measurement was completed, the quartz cell after washing was subjected to fluorescence measurement. As a result, red luminescence from the europium complex was reduced to a noise signal level, which indicated that the adhesion of the luminescent particles onto the wall surface was able to be suppressed.

Meanwhile, in Comparative Example 3, an increase in "r" was recognized from the 1st evaluation irrespective of the CRP antigen concentration.

At this time, no correlation was observed between the amount of change in "r" and the CRP concentration. Further, after the 10th measurement was completed, the quartz cell after washing was subjected to fluorescence measurement. As a result, strong red luminescence from the europium complex was observed, and the adhesion of the luminescent particles onto the wall surface of the cell was recognized.

It was indicated from the above-mentioned results that, in each of Examples, in which polyvinylpyrrolidone was added, and Reference Example 1, in which the wall surface of the cell was made of plastic, the formation of a hydrogen bond was not observed between the wall surface of the cell and the luminescent reagent, and the luminescent reagent did not adhere thereto. That is, it was indicated that polyvinylpyrrolidone serving as the hydrophilic polymer formed a hydrogen bond with a silanol group on the wall surface of the glass cell to suppress the adhesion of the luminescent reagent.

Table 1

| Item | Example 1 | Example 2 | Example 3 | Example 4 | Comparative Example 1 | Comparative Example 2 | Reference Example 1 |
|---|---|---|---|---|---|---|---|
| Cell | Quartz glass | Quartz glass | Quartz glass | Quartz glass | Quartz glass | Quartz glass | PMMA |
| Concentration of luminescent reagent (mg/mL) | 0.1 | 0.1 | 0.1 | 0.1 | 0.1 | 0.01 | 0.1 |
| Concentration of PVP in liquid (mass%) | 0.25 | 0.125 | 0.0625 | 0.03125 | Absent | Absent | Absent |
| Immersion time (hours) | 72 | 72 | 72 | 72 | 72 | 24 | 72 |
| Adhesion onto wall surface | Absent | Absent | Absent | Absent | Present | Present | Absent |

Table 2

| Concentration of CRP (pM) | 0 | 1 | 6.25 | 12.5 | 25 | 100 | 200 |
|---|---|---|---|---|---|---|---|
| Amount of change in "r" over 10 minutes (dr/dt) | 0.02 | 0.11 | 0.42 | 0.69 | 1.25 | 2.75 | 3.10 |

It was revealed from the foregoing that the measurement method according to each Example of the present invention was a method capable of repeatedly measuring the concentration of the CRP antigen serving as a target substance with high sensitivity while suppressing noise, such as the adhesion of the luminescent particles onto the glass cell.

Accordingly, when the measurement method according to each Example of the present invention is used, the presence or absence of the target substance or the concentration thereof can be stably measured with high sensitivity. It is conceived that the use of the measurement method according to each Example of the present invention can achieve an apparatus for stably performing measurement with high sensitivity in an application such as a specimen test in which mass testing is performed through repeated use of a glass cell.

According to the present invention, there can be provided an analysis method for calculating the concentration of a target substance through use of a reagent that reacts with the target substance, particularly a method, such as a method based on polarization anisotropy, capable of reducing noise by suppressing nonspecific adsorption of the reagent onto a glass vessel when high-sensitivity measurement is performed through use of a small amount of luminescent particles.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

**Claims**

1. An analysis method for determining at least any one of presence or absence of a target substance; and a concentration of the target substance through use of a reagent that reacts with the target substance, the analysis method comprising:

a loading step of loading: a sample containing the target substance; a hydrophilic polymer that reacts with a silanol group of a glass vessel; and the reagent into the glass vessel;
a reaction step of causing the target substance and the reagent to react with each other to provide a reaction liquid; and
an analysis step of determining at least any one of: the presence or absence of the target substance; and the concentration of the target substance in the reaction liquid,
wherein the reagent includes a luminescent reagent having a hydrophilic surface, and the luminescent reagent includes a luminescent particle, and
wherein the loading step includes loading the reagent after or simultaneously with the loading of the hydrophilic polymer.

2. The analysis method according to claim 1, wherein the analysis step includes determining at least any one of: the presence or absence of the target substance; and the concentration of the target substance in the reaction liquid by measuring a value (R) for polarization anisotropy of the reaction liquid.

3. The analysis method according to claim 2, wherein the following formula is satisfied: $R0 \geq 0.01$, where R0 represents the R measured for the reagent unreacted with the target substance.

4. The analysis method according to claim 2 or 3, wherein the R is defined to be "r" in the following equation (1):

$$r = \frac{I_{vv} - G * I_{vH}}{I_{vv} + 2 * G * I_{vH}} \qquad (1)$$
$$G = \frac{I_{Hv}}{I_{HH}}$$

in the equation (1),

Iw represents a luminescence intensity of a luminescence component having a vibration direction parallel to that of a first polarized light beam at a time of excitation by the first polarized light beam,
$I_{VH}$ represents a luminescence intensity of a luminescence component having a vibration direction orthogonal to that of the first polarized light beam at the time of excitation by the first polarized light beam,
$I_{HV}$ represents a luminescence intensity of a luminescence component having a vibration direction orthogonal to that of a second polarized light beam having a vibration direction orthogonal to that of the first polarized light beam at a time of excitation by the second polarized light beam,
$I_{HH}$ represents a luminescence intensity of a luminescence component having a vibration direction parallel to that of the second polarized light beam having a vibration direction orthogonal to that of the first polarized light beam at the time of excitation by the second polarized light beam, and
G represents a correction value.

5. The analysis method according to any one of claims 1 to 4, wherein the hydrophilic polymer forms a hydrogen bond with the silanol group of the glass vessel.

6. The analysis method according to any one of claims 1 to 5, wherein a concentration of the luminescent reagent in the reaction liquid is 0.0001 mg/ml or more and 0.1 mg/ml or less.

7. The analysis method according to any one of claims 1 to 6, wherein the hydrophilic polymer includes polyvinylpyrrolidone.

8. The analysis method according to any one of claims 1 to 7, wherein the luminescent particle contains a europium complex.

9. The analysis method according to any one of claims 1 to 8, wherein the luminescent reagent contains a ligand that binds to the target substance.

10. The analysis method according to claim 9, wherein the ligand is an antibody, and the target substance is an antigen.

11. An analysis apparatus for determining at least any one of presence or absence of a target substance; and a concentration of the target substance, the analysis apparatus comprising:

a glass vessel to be used for accommodating a reaction liquid containing: a sample containing the target substance; and a reagent that reacts with the target substance;

a unit configured to load the sample containing the target substance into the glass vessel;

a unit configured to load a hydrophilic polymer that reacts with a silanol group of the glass vessel into the glass vessel;

a unit configured to load the reagent into the glass vessel after or simultaneously with the loading of the hydrophilic polymer;

an irradiation unit configured to irradiate, as irradiation light, the glass vessel with light that is linearly polarized light;

a measurement unit configured to measure fluorescence intensities of fluorescence of two polarized light components in a parallel direction and an orthogonal direction with respect to the linearly polarized light; and

a processing unit configured to process results obtained by the measurement unit, to thereby determine at least any one of: the presence or absence of the target substance; and the concentration of the target substance in the reaction liquid,

wherein the reagent includes a luminescent reagent having a hydrophilic surface, and the luminescent reagent includes a luminescent particle.

12. An analysis kit to be used in an analysis method for determining at least any one of: presence or absence of a target substance; and a concentration of the target substance based on polarization anisotropy, the analysis kit comprising:

a reagent that reacts with the target substance;

a glass vessel to be used for accommodating a reaction liquid containing: a sample containing the target substance; and the reagent; and

a hydrophilic polymer that reacts with a silanol group of the glass vessel,

wherein the reagent includes a luminescent reagent having a hydrophilic surface, and the luminescent reagent includes a luminescent particle.

# FIG. 1

LOADING STEP

↓

REACTION STEP

↓

ANALYSIS STEP

↓

REMOVAL STEP

↓

WASHING STEP

FIG. 2

# FIG. 3

FIG. 4

# EUROPEAN SEARCH REPORT

**Application Number**

EP 24 19 8598

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/256595 A1 (LINK DARREN R [US] ET AL) 11 September 2014 (2014-09-11) | 1-4,6,9,10,12 | INV. G01N21/64 G01N15/00 |
| Y | * paragraphs [0013], [0014], [0077] - [0080], [0106], [0159], [0204], [0243] - [0278] * <br> * figures 1, 4, 7 * | 5,7,8 | ADD. G01N21/77 |
| X | US 5 627 522 A (WALKER DONNY R [US] ET AL) 6 May 1997 (1997-05-06) <br> * figures 1, 4, 62 * <br> * columns 19-24 * | 11 | |
| Y | CIACO STEFANO ET AL: "Inhibitors of UHRF1 base flipping activity showing cytotoxicity against cancer cells", BIOORGANIC CHEMISTRY, ACADEMIC PRESS INC., NEW YORK, NY, US, vol. 137, 22 May 2023 (2023-05-22), XP087335733, ISSN: 0045-2068, DOI: 10.1016/J.BIOORG.2023.106616 [retrieved on 2023-05-22] <br> * paragraph [04.2] * | 5 | |
| Y | WO 2022/259946 A1 (CANON KK [JP]; CANON MEDICAL SYSTEMS CORP [JP]) 15 December 2022 (2022-12-15) <br> * paragraph [0063] * | 8 | |

**TECHNICAL FIELDS SEARCHED (IPC)**

G01N

-/--

The present search report has been drawn up for all claims

| Place of search | Date of completion of the search | Examiner |
|---|---|---|
| Munich | 16 December 2024 | Brauer, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**Europäisches Patentamt**
**European Patent Office**
**Office européen des brevets**

# EUROPEAN SEARCH REPORT

**Application Number**

EP 24 19 8598

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KIM ET AL: "Fabrication and characterization of a PDMS-glass hybrid continuous-flow PCR chip", BIOCHEMICAL ENGINEERING JOURNAL, ELSEVIER, AMSTERDAM, NL, vol. 29, no. 1-2, 1 April 2006 (2006-04-01), pages 91-97, XP005335973, ISSN: 1369-703X, DOI: 10.1016/J.BEJ.2005.02.032 * paragraph [02.3] * | 7 | |
| A | US 2014/271369 A1 (FRITCHIE PATRICK P [US]) 18 September 2014 (2014-09-18) * the whole document * | 1-12 | |
| A | EP 0 355 738 A2 (HITACHI LTD [JP]) 28 February 1990 (1990-02-28) * the whole document * | 1-12 | |
| A | EP 0 197 425 A2 (ABBOTT LAB [US]) 15 October 1986 (1986-10-15) * the whole document * | 1-12 | TECHNICAL FIELDS SEARCHED (IPC) |

The present search report has been drawn up for all claims

| Place of search | Date of completion of the search | Examiner |
|---|---|---|
| Munich | 16 December 2024 | Brauer, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 4 521 099 A1

## ANNEX TO THE EUROPEAN SEARCH REPORT
## ON EUROPEAN PATENT APPLICATION NO.

EP 24 19 8598

16-12-2024

| Patent document cited in search report | | Publication date | Patent family member(s) | | Publication date |
|---|---|---|---|---|---|
| US 2014256595 | A1 | 11-09-2014 | AU | 2006335290 A1 | 19-07-2007 |
| | | | CA | 2636855 A1 | 19-07-2007 |
| | | | EP | 1984738 A2 | 29-10-2008 |
| | | | EP | 2363205 A2 | 07-09-2011 |
| | | | EP | 2364774 A2 | 14-09-2011 |
| | | | EP | 3913375 A1 | 24-11-2021 |
| | | | JP | 6163125 B2 | 12-07-2017 |
| | | | JP | 6317309 B2 | 25-04-2018 |
| | | | JP | 6723975 B2 | 15-07-2020 |
| | | | JP | 2009536313 A | 08-10-2009 |
| | | | JP | 2014138611 A | 31-07-2014 |
| | | | JP | 2016027827 A | 25-02-2016 |
| | | | JP | 2018046848 A | 29-03-2018 |
| | | | JP | 2018134096 A | 30-08-2018 |
| | | | US | 2010137163 A1 | 03-06-2010 |
| | | | US | 2013217583 A1 | 22-08-2013 |
| | | | US | 2014256595 A1 | 11-09-2014 |
| | | | US | 2014323317 A1 | 30-10-2014 |
| | | | US | 2017067047 A1 | 09-03-2017 |
| | | | US | 2018080020 A1 | 22-03-2018 |
| | | | US | 2018355350 A1 | 13-12-2018 |
| | | | WO | 2007081385 A2 | 19-07-2007 |
| | | | WO | 2007081386 A2 | 19-07-2007 |
| | | | WO | 2007081387 A1 | 19-07-2007 |
| US 5627522 | A | 06-05-1997 | NONE | | |
| WO 2022259946 | A1 | 15-12-2022 | CN | 117716228 A | 15-03-2024 |
| | | | EP | 4354119 A1 | 17-04-2024 |
| | | | JP | 2022187939 A | 20-12-2022 |
| | | | US | 2024118207 A1 | 11-04-2024 |
| | | | WO | 2022259946 A1 | 15-12-2022 |
| US 2014271369 | A1 | 18-09-2014 | NONE | | |
| EP 0355738 | A2 | 28-02-1990 | DE | 68919165 T2 | 09-03-1995 |
| | | | EP | 0355738 A2 | 28-02-1990 |
| | | | JP | H0259647 A | 28-02-1990 |
| | | | JP | H0758263 B2 | 21-06-1995 |
| EP 0197425 | A2 | 15-10-1986 | EP | 0197425 A2 | 15-10-1986 |
| | | | JP | S61274243 A | 04-12-1986 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H0352575 B

- JP 2893772 B